# EUROPEAN PATENT APPLICATION

(11) **EP 4 847 689 A1**
(43) Date of publication of application: **02.12.2026**
(21) Application number: 25179375.8
(22) Date of filing: 28.05.2025
(51) Int. Cl.: D06M 16/00, D06M 15/03

(54) **MULTILAYER COATING METHOD AND APPARATUS FOR MEDICAL TEXTILES**

(71) Applicant: Paul Hartmann AG, 89522 Heidenheim (DE)
(72) Inventor: SCHMIDT, Christian, 73466 Lauchheim (DE)
(74) Representative: Paul Hartmann AG Patents & Licensing

(57) **Abstract**

The present disclosure provides a method for coating a medical textile substrate with at least two alternating layers of a coating. The method includes providing a textile substrate, applying a coating solution A comprising coating molecules A to the textile substrate, removing excessive residues of coating solution A, applying a coating solution B comprising coating molecules B to the textile substrate, and removing excessive residues of coating solution B. The coating molecules A and B can attract each other through electrostatic attraction. An apparatus for coating a textile substrate web includes an unwinder roller (1) or donor container, a first coating bath (3) or spray-coating device, a first rinsing bath (5) or flushing device, a second coating bath (6) or spray-coating device, a second rinsing bath (7) or flushing device, a drying device (10), a rewinder roller (12) or recipient container, and diverter rollers (20) or bars for guiding the textile substrate web (15) through the process. A medical dressing or bandage comprises a coated textile layer cut out of the coated textile substrate.

## Description

### FIELD OF INVENTION

The present invention relates to methods and apparatuses for coating textile substrates, and more particularly to a method and apparatus for producing antimicrobial multilayer coatings on medical dressings or bandages using an industrial-scale process.

### BACKGROUND

Textile materials are widely used in various applications, including medical and healthcare settings. Antimicrobial coatings on textiles can provide protection against harmful microorganisms and improve hygiene. Applying such coatings to textile substrates presents challenges in achieving uniform coverage, durability, and maintaining the fabric's original properties.

One approach to coating textiles involves immersing the fabric in a solution containing the desired coating material. US5409739 A describes a process for producing a crosslinked monolayer coating on a substrate using block copolymers. This method involves introducing a block copolymer into a selective solvent, contacting the substrate with the resulting semi-solution, and crosslinking the coating. While this approach can create thin, durable coatings, it may not be suitable for applying multiple layers or for continuous processing of large textile webs.

WO 2016/011944 A1 discloses a multi-layer coating system comprising two different coating compositions applied sequentially. The system aims to provide good adhesion and resistance properties. However, this method focuses on coating rigid substrates like plastics for electronic devices and may not be directly applicable to flexible textile materials or continuous web processing.

WO 2017/191110 A1 discloses a method for coating a substrate with alternating layers of polyelectrolytes. The document describes using this technique to create antimicrobial coatings on medical devices and implants. The method as described does not disclose coating of flexible textile substrates used in medical dressings or bandages. Also, while this approach allows for the creation of multilayer coatings with controlled thickness, it may face challenges in scaling up for continuous processing of large textile webs at industrial speeds.

US4093757 A presents a method for coating a substrate with a monomolecular layer of organic molecules using a Langmuir-Blodgett technique. This approach allows for precise control of layer thickness but may be limited in terms of processing speed and scalability for large textile webs.

US 2020/0223201 A1 describes a method for treating substrates using atmospheric plasma to affix biomaterials like silk or wool polypeptides to textile surfaces. While this technique offers advantages in terms of environmental friendliness, it may not be suitable for applying multiple distinct layers of coating materials.

US6762243 B2 discloses a multilayer coating system using polyurethane compositions. This system provides flexibility and durability but may not offer the precision required for creating alternating layers of different coating materials on a textile substrate.

CN106637925 A presents a method for preparing ultraviolet-resistant cotton fabric using a single-molecule layer of graphene oxide. While this approach demonstrates the potential for thin, functional coatings on textiles, it is limited to a single layer and specific coating material.

Existing coating methods for textiles face several significant challenges that limit their effectiveness, particularly for antimicrobial applications in medical and healthcare settings:
1. Difficulty in applying multiple distinct layers of coating materials - Current methods typically focus on single-layer applications and struggle to create well-defined, alternating layers of different coating materials with precise interfaces between them. This limitation restricts the ability to create coatings with complementary properties or synergistic antimicrobial effects.
2. Limited control over layer thickness and uniformity - Conventional coating techniques often result in inconsistent layer thickness across the textile substrate, with variations that can exceed 50% between different areas of the same substrate. This non-uniformity leads to unpredictable performance characteristics and potential weak points in antimicrobial protection.
3. Challenges in continuous processing of large textile webs - Many existing coating methods are batch processes or limited to small-scale applications, making them impractical for industrial-scale production of coated textiles. Continuous web processing at speeds sufficient for commercial viability (typically 5-20 meters per minute) presents significant technical hurdles for maintaining coating quality and consistency.
4. Potential alteration of textile properties like flexibility and breathability - Many coating processes significantly compromise the inherent properties of the textile substrate, reducing breathability by for example up to 40-60%, increasing stiffness, and altering tactile characteristics. These changes can render the coated textiles unsuitable for applications requiring comfort and flexibility, such as medical garments or wound dressings.
5. Limited scalability for industrial production - Techniques such as Langmuir-Blodgett deposition or plasma treatment offer excellent control but are difficult to scale up economically. The capital investment, energy consumption, and operational complexity of these methods often make them prohibitively expensive for large-scale textile production, with costs typically 3-5 times higher than conventional textile finishing processes.
6. Inadequate durability of coatings, especially for medical applications - Many antimicrobial coatings show significant degradation after relatively few washing cycles (typically losing 30-50% efficacy after 5-10 washes) or during sterilization processes. This limited durability restricts their use in medical textiles that require repeated cleaning or sterilization while maintaining consistent antimicrobial performance throughout their service life.
7. Insufficient or inconsistent antimicrobial activity, particularly after repeated washing or extended use - Single-layer antimicrobial coatings often exhibit diminishing efficacy over time, with significant reductions in antimicrobial performance (measured by log reduction values against test organisms) after exposure to moisture, bodily fluids, or cleaning agents. This inconsistency creates unpredictable protection levels and potential failure points in critical applications where sustained antimicrobial activity is essential.
8. Difficulty to generate an essentially monomolecular layer of coating molecules.

Considering these limitations, there is a need for an improved method and apparatus for applying multiple layers of functional coatings to textile substrates in a continuous, scalable process while maintaining the desirable properties of the base material.

### DETAILED DESCRIPTION

The present invention solves the problems mentioned above by providing a method and apparatus for producing alternating coatings quickly, easily, and cost-effectively on an industrial scale. The invention enables the application of alternating layers, in particular alternating monomolecular layers of coating agents on medical textiles, achieving a resource-efficient process without requiring additional environmental conditions such as vacuuming.

It was surprisingly found that alternating layers of coatings can be formed on a textile substrate, where two different types of coating molecules A and coating molecules B attract each other through electrostatic attraction. This discovery may provide several advantages for creating functional coatings on medical textiles.

The electrostatic attraction between coating molecules A and B may contribute to the formation of stable and well-defined multilayer structures on the textile substrate surface. This interaction may allow for the creation of coatings with precise control over layer composition and thickness.

In some aspects, the electrostatic attraction between alternating layers may enhance the overall stability and durability of the coating. The strong interactions between oppositely charged molecules may help resist delamination or degradation of the coating during use or washing.

The alternating layer structure formed through electrostatic attraction may allow for the incorporation of different functional molecules in each layer. This approach may enable the creation of multifunctional coatings of medical textile materials with synergistic properties, such as combined antimicrobial and moisture management capabilities.

In some cases, the electrostatic attraction between layers may contribute to improved coating uniformity across the textile substrate. The self-assembly nature of the process, driven by electrostatic interactions, may help ensure consistent coverage even on complex textile structures or large surface areas.

The ability to form alternating layers through electrostatic attraction may simplify the coating process of textile substrates compared to methods requiring additional crosslinking steps or complex chemical reactions. This simplification may contribute to improved scalability and cost-effectiveness in industrial production settings.

The method involves coating a medical textile substrate with at least two alternating layers of a coating. The process includes providing the textile substrate, applying a coating solution A comprising coating molecules A to the textile substrate, removing excessive residues of the coating solution A, applying a coating solution B comprising coating molecules B to the textile substrate, removing excessive residues of the coating solution B, and optionally drying the textile substrate. The coating molecules A and the coating molecules B can attract each other through electrostatic attraction.

An apparatus for coating a textile substrate web is also provided. The apparatus comprises an unwinder roller or donor container for providing the textile substrate web, a first coating bath or spray-coating device, a first rinsing bath or flushing device, a second coating bath or spray-coating device, a second rinsing bath or flushing device, a drying device, a rewinder roller or recipient container for collecting the coated textile substrate web, and a plurality of diverter rollers or diverter bars for guiding the textile substrate web through the process.

The invention provides a versatile and efficient method for applying multilayer coatings to medical textile substrates. By utilizing different coating solutions and implementing a continuous process, the method allows for the creation of complex coating structures with precise control over layer composition and thickness. The removal of excessive residues between coating applications ensures clean and well-defined layers, while the optional drying step can be used to optimize coating adhesion and performance.

This coating method offers several advantages, including the ability to create antimicrobial coatings for medical applications, improved control over coating properties, and the flexibility to use a wide range of coating materials. The continuous nature of the process allows for high-volume production while maintaining consistent coating quality.

The present invention solves the problems mentioned above by providing a method for coating a medical textile substrate with at least two alternating layers of a coating. The method comprises the following steps:
The method comprises the steps of providing a medical textile substrate; applying a coating solution A comprising coating molecules A to the medical textile substrate; removing excessive residues of the coating solution A from the medical textile substrate; applying a coating solution B comprising coating molecules B to the medical textile substrate; removing excessive residues of the coating solution B from the medical textile substrate; and optionally drying the medical textile substrate. The coating molecules A and the coating molecules B can attract each other through electrostatic attraction.

The method enables the application of alternating layers, in particular alternating monomolecular layers of coating agents on medical textiles. This process achieves a resource-efficient coating without requiring additional environmental conditions such as vacuuming.

In the first step, a medical textile substrate is provided. The medical textile substrate may be a woven, knitted, or nonwoven fabric suitable for medical applications such as wound dressings, bandages, or surgical textiles.

The second step involves applying coating solution A to the medical textile substrate. Coating solution A contains coating molecules A, which may be positively charged polyelectrolytes such as Poly-L-arginine or Poly-ε-Lysine. The application of coating solution A may be performed by immersion, spraying, or other suitable coating techniques.

In the third step, excessive residues of coating solution A are removed from the medical textile substrate. This removal may be accomplished through rinsing, squeezing, or a combination of these methods. The removal of excess coating solution ensures a uniform and controlled coating layer.

The fourth step involves applying coating solution B to the medical textile substrate. Coating solution B contains coating molecules B, which may be negatively charged polyelectrolytes such as hyaluronan. The application method for coating solution B may be similar to that used for coating solution A.

In the fifth step, excessive residues of coating solution B are removed from the medical textile substrate. This removal process may be similar to the one used for coating solution A, ensuring a clean and well-defined coating layer.

The optional sixth step involves drying the medical textile substrate. Drying may be performed using various methods such as air drying, forced air drying, or heat drying, depending on the specific requirements of the coating materials and the medical textile substrate.

The electrostatic attraction between coating molecules A and coating molecules B plays a crucial role in the formation of stable, in particular monomolecular, multilayer coatings. The oppositely charged molecules form strong electrostatic bonds, resulting in self-assembled polyelectrolyte multilayers on the surface of the medical textile substrate. The formation of essentially monomolecular layers is further promoted by the fact that the molecules A may repel each other due to their ionic character and that also the molecules B may repel each other due to their ionic character.

This method may be repeated multiple times to build up additional alternating layers on the medical textile substrate. The number of repetitions may be adjusted based on the specific requirements of the final coated textile product, typically ranging from 2 to 100 cycles, preferably between 4 and 30 cycles.

The duration of each coating application step may be optimized for efficient processing while ensuring adequate coating formation. Typically, applying each of the coating solutions A and B to the medical textile substrate may be performed between 3 seconds and 300 seconds, in particular between 10 seconds and 30 seconds.

This coating method offers several advantages for medical textile applications. The alternating layer structure allows for precise control over coating thickness and composition. The electrostatic interactions between layers contribute to coating stability and durability. The method may be used to create antimicrobial coatings, controlled drug release systems, or other functional coatings for medical textiles.

The versatility of this coating method allows for the use of various coating materials beyond Poly-L-arginine, Poly-ε-Lysine, and hyaluronan. Other biocompatible polyelectrolytes or functional molecules may be incorporated into the coating solutions to achieve specific properties or functionalities in the coated medical textile substrate.

The coating method described in the present invention provides antimicrobial properties to the medical textile substrate. The antimicrobial effectiveness of the coating enhances the functionality of the medical textile substrate by reducing the risk of bacterial colonization and infection.

The antimicrobial properties of the coating may be attributed to the specific materials used in the coating process. For example, when Poly-L-arginine or Poly-ε-Lysine is used as coating molecules A, these cationic polymers may exhibit inherent antimicrobial activity. The positively charged amino groups of these polymers may interact with negatively charged bacterial cell membranes, leading to membrane disruption and bacterial cell death.

The multilayer structure of the coating may contribute to sustained antimicrobial activity. As the coating layers gradually degrade or dissolve, fresh antimicrobial components may be exposed, providing long-lasting protection against microbial growth on the medical textile substrate.

The antimicrobial coating may be effective against a broad spectrum of microorganisms, including gram-positive bacteria, gram-negative bacteria, and certain fungi. This broad-spectrum activity may make the coated medical textile substrate suitable for various medical applications where infection control is a concern.

In some examples, the antimicrobial effectiveness of the coating may be further enhanced by incorporating additional antimicrobial agents into the coating solutions. These agents may include silver nanoparticles, quaternary ammonium compounds, or other biocompatible antimicrobial substances. The multilayer coating structure may allow for controlled release of these antimicrobial agents, providing sustained protection over time.

The antimicrobial properties of the coated medical textile substrate may be particularly beneficial in wound care applications. The coating may help prevent bacterial colonization of the wound site, reducing the risk of infection and promoting faster healing. Additionally, the antimicrobial coating may extend the usable life of the medical textile substrate by preventing microbial growth during storage and use.

The effectiveness of the antimicrobial coating may be evaluated through standard microbiological testing methods. These tests may include zone of inhibition assays, time-kill studies, and bacterial adhesion tests. The results of such tests may demonstrate the ability of the coated medical textile substrate to inhibit bacterial growth and reduce microbial colonization.

The antimicrobial properties of the coating may be tailored to specific applications by adjusting the composition and number of coating layers. For example, a higher number of antimicrobial layers may be applied for medical textiles intended for use in high-risk environments or for extended periods.

In addition to its antimicrobial properties, the coating may also exhibit anti-biofilm characteristics. The multilayer structure and surface properties of the coating may inhibit the formation of bacterial biofilms on the medical textile substrate. This anti-biofilm effect may further enhance the infection control capabilities of the coated medical textile.

The combination of antimicrobial and anti-biofilm properties may make the coated medical textile substrate particularly suitable for applications such as wound dressings, surgical drapes, and medical device covers. These properties may help reduce the risk of healthcare-associated infections and improve patient outcomes.

The coating materials used in the present invention play a crucial role in creating functional multilayer coatings on medical textile substrates. The primary coating materials include Poly-L-arginine, Poly-ε-Lysine, and hyaluronan, which are selected for their biocompatibility and ability to form stable electrostatic interactions.

Coating solution A typically contains Poly-L-arginine or Poly-ε-Lysine as the primary coating molecule. Poly-L-arginine is a cationic polypeptide composed of L-arginine amino acid residues linked by peptide bonds. Poly-ε-Lysine is a cationic homopolymer composed of L-lysine amino acid residues. Both polymers carry positive charges at physiological pH due to their amino groups.

The concentration of Poly-L-arginine in coating solution A can be 0.1 to 2.0 mg/mL. This concentration range allows for effective formation of monomolecular layers while maintaining process efficiency. Coating solution A can have a pH of 6.0 to 7.5, which ensures that the Poly-L-arginine or Poly-ε-Lysine remains positively charged, promoting electrostatic interactions with the substrate and subsequent layers.

Coating solution B typically contains hyaluronan (also known as hyaluronic acid) as the primary coating molecule. Hyaluronan is an anionic, non-sulfated glycosaminoglycan distributed widely throughout connective, epithelial, and neural tissues. The Hyaluronan in coating solution B can have a molecular weight of 1.0 to 1.5 MDa. This molecular weight range provides a good balance between solution viscosity, layer formation properties, and biological activity.

The concentration of Hyaluronan in coating solution B can be 0.1 to 2.0 mg/mL, more preferably 0.5 mg/ml. This concentration range allows for effective formation of monomolecular layers while maintaining process efficiency. Coating solution B can have a pH similar to coating solution A, typically in the range of 6.0 to 7.5.

The solvent used for coating solutions A and B can be a NaCl-Tris buffer comprising 150 mM NaCl and 10 mM Tris at a pH 7.4. This buffer provides a controlled environment for the dissolution of the polyelectrolytes and helps maintain the desired pH of the coating solutions, ensuring consistent performance throughout the coating process.

The formation of monomolecular layers is a preferred feature of this coating process. When the textile substrate is exposed to coating solution A, and excessive residues of coating solution A are removed, a monomolecular layer of Poly-L-arginine or Poly-ε-Lysine is formed on the substrate surface. Similarly, when the substrate is exposed to coating solution B, and excessive residues of coating solution B are removed, a monomolecular layer of hyaluronan is formed. These ultra-thin layers allow for precise control over the coating structure and properties.

The term "monomolecular layer" refers to a coating layer that is predominantly one molecule thick. While some minor deviations from a perfect single-molecule arrangement may occur due to factors such as surface irregularities or molecular interactions, the overall coating maintains the characteristics and benefits of a monomolecular structure.

The formation of monomolecular layers offers several advantages. First, monomolecular layers allow for extremely precise control over coating thickness, as each layer adds only a few nanometers to the overall coating. This level of control enables the creation of coatings with specific functional properties tailored to the intended application.

Second, the monomolecular nature of each layer promotes strong electrostatic interactions between adjacent layers, contributing to the overall stability and durability of the coating. The alternating positive and negative charges of the Poly-L-arginine/Poly-ε-Lysine and hyaluronan layers create a tightly bound multilayer structure.

Third, monomolecular layers may allow for more efficient use of coating materials, as each layer requires only a small amount of material to cover the entire substrate surface. This efficiency may contribute to the cost-effectiveness and sustainability of the coating process.

Fourth, the monomolecular structure of each layer may facilitate uniform coverage of complex substrate geometries, including porous or fibrous textile structures. The thin nature of each layer allows the coating to conform closely to the substrate surface, potentially enhancing coating adhesion and performance.

In summary, the use of Poly-L-arginine (or Poly-ε-Lysine) together with hyaluronan as coating materials, combined with the formation of monomolecular layers, enables the creation of precisely controlled, stable, and functional multilayer coatings on medical textile substrates. The specific concentrations, molecular weights, and buffer conditions described provide a framework for optimizing the coating process for various applications in the medical textile field.

The coating process may preferably be repeated multiple times to build up additional layers on the medical textile substrate. In some examples, the method involves cycling the substrate through the coating and rinsing steps several times to achieve the desired number of alternating layers. The number of repetitions may be adjusted based on the specific requirements of the final coated textile product.

By repeating the process, multiple bilayers consisting of coating A and coating B may be deposited on the substrate surface. In some cases, this repetition may allow for the creation of complex multilayer structures with enhanced functional properties. The ability to control the number of coating cycles may provide flexibility in tailoring the thickness, composition, and performance characteristics of the final coated textile.

The repeated application of alternating layers may contribute to improved coating durability, as each additional layer may reinforce the overall coating structure. In some implementations, the multilayer coating formed through repeated cycles may exhibit synergistic effects between the different coating materials, potentially leading to enhanced antimicrobial efficacy, improved barrier properties, or other desired functionalities.

Preferably, the method may include repeating cycles comprising the coating and rinsing steps between 2 and 100 times, preferably between 4 and 30 times. This range of repetitions may allow for the creation of between 4 and 200, preferably between 8 and 60 alternating coating layers on the medical textile substrate.

The optimal number of coating cycles may depend on various factors, including the intended application of the coated textile, the desired thickness of the coating, and the specific properties required. For example, a higher number of coating cycles may be beneficial for applications requiring enhanced antimicrobial activity or improved barrier properties. Conversely, fewer coating cycles may be sufficient for applications where a thinner coating is desired or where rapid processing time is a priority.

Repeated coating cycles may offer several advantages:
1. Increased coating thickness: Each cycle adds to the overall thickness of the coating, allowing for precise control over the final coating dimensions.
2. Enhanced functionality: Additional layers may amplify the functional properties of the coating, such as antimicrobial activity or moisture management capabilities.
3. Improved uniformity: Multiple coating cycles may help to ensure more uniform coverage of the substrate, particularly for textiles with complex structures or surface irregularities.
4. Customization: The number of cycles can be adjusted to tailor the coating properties for specific applications or performance requirements.
5. Layered functionality: Different coating solutions can be used in alternating cycles to create multi-functional coatings with distinct properties in each layer.

In some examples, the coating process may be extended to include additional coating solutions containing different coating molecules. This approach may result in alternating sets of three, four or more different layers on the medical textile substrate. For instance, a third coating solution C with coating molecules C, and a fourth coating solution D with coating molecules D may be incorporated into the coating sequence.

The method may involve applying coating solution A, followed by coating solution B, then coating solution C, and optionally coating solution D, in a repeating cycle. Between each coating application, the excess coating solution may be removed, and the substrate may undergo a rinsing step to prepare for the next coating layer.

This multi-layer approach may allow for the creation of more complex coating structures with enhanced functionality. For example, each layer may contribute different properties to the final coated textile:
- Layer A may provide antimicrobial anr/or anti-biofilm properties
- Layer B may enhance durability
- Layer C may improve moisture management
- Layer D may add a specific functional property such as UV protection or flame retardancy

The alternating nature of these multi-layer coatings may result in synergistic effects between the different coating molecules. For example, the interaction between adjacent layers may lead to improved overall coating stability or the creation of unique surface properties that are not achievable with single or dual-layer coatings.

The number of coating cycles may be adjusted to achieve the desired coating thickness and performance characteristics. The process may be repeated to create multiple sets of these alternating layers, with each set potentially consisting of three or four distinct layers.

This multi-layer coating approach may offer increased versatility in tailoring the properties of the coated medical textile substrate for specific applications. The method may allow for the development of advanced functional textiles with a combination of properties that are particularly well-suited for complex medical or technical applications.

The duration of coating application plays a crucial role in the efficiency and quality of the coating process. Applying each of the coating solutions A and B to the textile substrate web may be performed between 3 seconds and 300 seconds, in particular between 10 seconds and 30 seconds. This range allows for adequate coating application while maintaining efficient processing speeds.

The speed at which the textile substrate travels through the coating apparatus ranges from 0.1 m/min to 100 m/min, with a preferred range of 1 m/min to 50 m/min. This speed range allows for efficient production while ensuring adequate coating application and processing time.

Slower speeds within this range may result in thicker coatings, as the textile substrate spends more time in contact with the coating solutions. This extended contact time may allow for deeper penetration of the coating into the textile structure, potentially enhancing coating adhesion and durability.

Conversely, faster speeds may lead to thinner, more uniform coatings. Higher processing speeds may be advantageous for large-scale production, allowing for increased throughput and reduced processing time. However, care must be taken to ensure that the coating application remains sufficient at higher speeds.

The optimal speed and coating application time may depend on various factors, including the specific textile substrate properties, the viscosity of the coating solutions, and the desired coating thickness. Adjusting these parameters allows for fine-tuning of the coating process to achieve the desired balance between production efficiency and coating quality.

In some examples, the coating application time and processing speed may be adjusted based on the number of coating layers being applied. For instance, when applying a higher number of alternating layers, slightly faster processing speeds or shorter coating application times may be used for each individual layer to maintain overall production efficiency.

The ability to control coating application time and processing speed provides flexibility in adapting the coating process to different textile substrates and coating formulations. This adaptability allows for optimization of the coating process for various medical textile applications, ensuring consistent and high-quality coatings across a range of products.

The coating method described in the present invention may be applied to various types of textile substrates suitable for medical applications. The textile substrate may comprise one or more of woven textiles, knitted textiles, nonwoven textiles, and textile nets.

Woven textiles are produced by interlacing two sets of yarns, typically at right angles to each other. The warp yarns run lengthwise in the fabric, while the weft yarns run across the width. Woven textiles offer excellent dimensional stability and strength, making them suitable for applications requiring durability. The regular structure of woven fabrics may provide a uniform surface for coating application, potentially resulting in consistent coating coverage.

Knitted textiles are created by interlocking loops of yarn. Knitted fabrics may be categorized into weft-knitted and warp-knitted structures. Knitted textiles typically offer greater flexibility and stretch compared to woven fabrics. This elasticity may be advantageous for certain medical applications, such as compression bandages or form-fitting wound dressings. The porous nature of knitted structures may allow for enhanced penetration of coating solutions, potentially resulting in improved coating adhesion.

Nonwoven textiles are made from fibers bonded together by chemical, mechanical, heat, or solvent treatment. Nonwoven fabrics may offer a combination of properties from both woven and knitted structures. These textiles may provide excellent absorbency, softness, and conformability, making them suitable for applications such as disposable medical gowns or wound dressings. The fibrous structure of nonwoven textiles may create a large surface area for coating application, potentially enhancing the functional properties of the coated substrate.

Textile nets are open-mesh fabrics with a grid-like structure. These textiles may be produced through various methods, including weaving, knitting, or extrusion. Textile nets may offer high porosity and breathability, making them suitable for applications requiring air and moisture permeability. The open structure of textile nets may allow for efficient coating application and may facilitate the creation of coatings with specific functional properties.

The textile substrate may have an open-pored structure with a pore diameter of 0.1 to 5 mm. This pore size range may influence the coating process and the properties of the final coated textile. Smaller pores (0.1-1 mm) may result in more surface-concentrated coatings with limited penetration into the substrate core. Larger pores (1-5 mm) may allow deeper penetration of coating solutions, potentially resulting in more uniform distribution throughout the textile thickness.

The choice of textile substrate type may depend on the specific requirements of the intended medical application. Factors such as strength, flexibility, absorbency, breathability, and biocompatibility may influence the selection of the appropriate textile substrate for coating. The coating method described in this invention may be adapted to accommodate the unique characteristics of each textile substrate type, allowing for the creation of functional coated textiles tailored to specific medical needs.

The textile substrate used in the coating process has an open-pored structure that plays a significant role in the coating application and final properties of the coated medical textile. The open-pored structure allows for effective penetration and distribution of the coating solutions throughout the textile substrate.

The porosity of the textile substrate ranges from 10% to 90%, with a preferred range of 50% to 80%. This porosity range affects the interaction between the coating solutions and the substrate structure. Higher porosity allows for better penetration of the coating solutions into the textile structure, while lower porosity may result in more surface-concentrated coatings.

The open-pored structure of the textile substrate offers several advantages in the coating process:
1. Enhanced coating penetration: The porous nature of the substrate allows the coating solutions to penetrate deeper into the textile structure, potentially resulting in more uniform coating distribution throughout the substrate thickness.
2. Increased surface area: The open pores provide a larger surface area for coating adhesion, which may enhance the overall coating effectiveness and functional properties of the coated textile.
3. Improved coating stability: The porous structure may allow for mechanical interlocking of the coating within the textile substrate, potentially improving the durability and adhesion of the coating.
4. Controlled release properties: The porous nature of the substrate may facilitate controlled release of antimicrobial or other functional agents incorporated in the coating, as the pores can act as reservoirs for these substances.
5. Maintained breathability: The open-pored structure helps maintain the breathability of the textile substrate even after coating application, which may be beneficial for certain medical applications requiring air and moisture permeability.
6. Customizable coating distribution: By selecting substrates with different porosity levels within the specified range, the coating distribution and properties can be tailored for specific medical applications.

The porosity of the textile substrate may be achieved through various manufacturing methods, depending on the type of textile (woven, knitted, nonwoven, or textile net). For woven and knitted textiles, the porosity can be controlled by adjusting the yarn spacing and structure. In nonwoven textiles, porosity can be influenced by fiber density and bonding techniques. For textile nets, the mesh size and structure determine the overall porosity.

The selection of the appropriate porosity level within the specified range depends on the intended application of the coated medical textile. For example, higher porosity levels may be preferred for applications requiring rapid fluid absorption or high breathability, while lower porosity levels may be suitable for applications needing enhanced barrier properties or surface-concentrated antimicrobial activity.

The present invention provides multiple methods for applying coating solutions to medical textile substrates, including immersion in coating baths and spray-coating techniques. These methods allow for efficient and uniform application of coating materials on an industrial scale.

Immersion coating involves submerging the medical textile substrate in a coating bath containing the desired coating solution. A first coating bath may be used to apply coating solution A, while a second coating bath may be used to apply coating solution B. The immersion process allows for thorough penetration of the coating solution into the textile structure, ensuring comprehensive coverage of individual fibers.

During the immersion process, the medical textile substrate may be guided through a first coating bath containing coating solution A. After the application of coating solution A, the substrate may pass through a first rinsing bath to remove excess coating material. Subsequently, the substrate may be guided through a second coating bath containing coating solution B, followed by a second rinsing bath to remove excess coating solution B.

The coating solutions used in the immersion process have a viscosity ranging from 0.25 cP to 1000 cP, with a preferred range of 0.30 cP to 2.00 cP. This viscosity range facilitates proper coating application and penetration into the textile structure. Lower viscosities may allow for better penetration into porous substrates, while higher viscosities may result in thicker coating layers.

The temperature of the coating baths is maintained within a range of 10°C to 80°C, with a preferred range of 20°C to 60°C. This temperature range helps optimize the coating process for different coating materials and substrate types. Higher temperatures may increase the rate of layer formation but may also affect the stability of certain coating materials.

Spray-coating techniques offer an alternative method for applying coating solutions to medical textile substrates. In this approach, coating solutions A and B may be applied using spray-coating devices. Spray-coating allows for precise control over the amount of coating solution applied and may be particularly useful for selectively coating specific areas of the textile substrate.

The spray-coating process may involve applying coating solution A using a first spray-coating device, followed by the application of coating solution B using a second spray-coating device. Between coating applications, rinsing or flushing steps may be implemented to remove excess coating material and prepare the substrate for the next layer.

Both immersion and spray-coating methods offer distinct advantages and may be selected based on the specific requirements of the medical textile substrate and the desired coating properties. Immersion coating may provide superior penetration into the textile structure, while spray-coating may offer greater control over coating distribution and thickness.

The coating application methods described in this invention allow for the creation of uniform and functional coatings on medical textile substrates, enabling the development of advanced materials for various medical applications.

The present invention provides a method for continuous processing of a long textile substrate web, enabling efficient and large-scale production of coated medical textiles. This continuous process allows for the application of multiple coating layers while maintaining consistent quality throughout the length of the textile substrate web.

The textile substrate web is provided on an unwinder roller. The textile substrate web has a web width of 110 cm, which is a typical dimension for industrial coating processes. This width allows for efficient use of standard coating equipment while providing sufficient material for various end-use applications. The textile substrate web has a web length of 50 to 200 m, allowing for extended production runs without frequent changeovers.

The textile substrate web is transported from the unwinder roller to a rewinder roller in a continuous manner. This continuous movement enables uninterrupted coating application and processing. The transport mechanism utilizes diverter devices, particularly diverter rollers, to guide the textile substrate web through various processing stations.

Diverter rollers are positioned at strategic points along the processing path to maintain proper tension and alignment of the textile substrate web. These diverter rollers help control the path of the textile substrate web, ensuring consistent contact with coating solutions and even distribution of coatings across the substrate surface.

The continuous processing method allows for the application of multiple coating layers in sequence without the need for intermediate winding and unwinding steps. As the textile substrate web moves through the apparatus, coating solutions can be applied, excess coating removed, and rinsing steps performed in a continuous flow.

The use of an unwinder roller and a rewinder roller facilitates the handling of long lengths of textile substrate web. The unwinder roller provides a controlled release of the uncoated textile substrate web into the processing apparatus, while the rewinder roller collects the coated textile substrate web at the end of the process.

This continuous processing approach offers several advantages for large-scale production of coated medical textiles. The method allows for high-volume output while maintaining consistent coating quality throughout the length of the textile substrate web. The continuous nature of the process reduces handling and potential contamination risks associated with batch processing methods.

The transport mechanism using diverter devices enables precise control over the textile substrate web's path through the coating apparatus. This control helps maintain proper tension and alignment, which are crucial for achieving uniform coating application and preventing defects in the final coated product.

The continuous processing method can be adapted to accommodate various coating sequences and processing steps. Additional coating baths or spray-coating devices can be incorporated into the processing line to apply multiple layers or different types of coatings in a single pass through the apparatus.

The present invention solves the problems mentioned above by providing an efficient and effective method for removing excessive residues of coating solutions from the medical textile substrate. This residue removal process is a crucial step in ensuring the quality and uniformity of the applied coating layers.

After applying a coating solution to the medical textile substrate, excessive residues are removed to prevent uneven coating thickness and potential interference with subsequent coating layers. The residue removal process can be performed using rinsing baths or flushing devices.

A first rinsing bath may be used to remove excessive residues of coating solution A from the medical textile substrate. The first rinsing bath contains a buffer solution that helps neutralize and remove any unbound coating molecules from the substrate surface. The medical textile substrate may be immersed in the first rinsing bath for a predetermined duration to ensure thorough removal of excess coating material.

Similarly, a second rinsing bath may be employed to remove excessive residues of coating solution B from the medical textile substrate. The second rinsing bath also contains a buffer solution tailored to effectively remove unbound molecules of coating solution B. The use of separate rinsing baths for each coating solution helps prevent cross-contamination and maintains the integrity of each coating layer.

In some examples, flushing devices may be used instead of or in addition to rinsing baths. These flushing devices may apply a controlled stream of buffer solution to the surface of the medical textile substrate, effectively removing excess coating material. Flushing devices may offer advantages in terms of reduced water consumption and more targeted residue removal.

The duration of the residue removal process may be optimized based on factors such as the type of coating solution, substrate material, and desired coating properties. Typically, the rinsing or flushing step may last between 5 and 60 seconds, preferably about 30 seconds, with the exact duration determined through experimentation and process optimization.

The buffer solutions used in the rinsing baths or flushing devices may be formulated to match the pH and ionic strength of the coating solutions. This formulation helps maintain the stability of the applied coating layer while effectively removing excess material. The composition of the buffer solutions may be adjusted based on the specific coating materials and substrate properties.

In some examples, the residue removal process may incorporate gentle agitation or movement of the medical textile substrate within the rinsing bath. This agitation can enhance the removal of excess coating material, particularly from porous or textured substrate surfaces.

The temperature of the rinsing baths or flushing solutions may be controlled to optimize the residue removal process. The temperature may typically range from 15°C to 90°C, preferably between 18°C and 60°C, particularly preferred between 20°C and 30°C with the specific temperature selected based on the properties of the coating materials and substrate.

The temperature of the rinsing baths or flushing solutions may be controlled to optimize the residue removal process. While the temperature may typically range from 15°C to 90°C, room temperature (approximately 20-25°C) may be preferred for several reasons. Operating the rinsing process at room temperature may be easier to accomplish in many manufacturing environments, as it does not require additional heating or cooling equipment. This approach may also contribute to energy savings, as maintaining the rinsing solutions at room temperature generally consumes less energy compared to heating or cooling the solutions. Additionally, room temperature processing may be suitable for a wide range of coating materials and substrates, potentially offering a versatile solution for various medical textile applications. However, the specific temperature may still be selected based on the properties of the coating materials and substrate to ensure optimal residue removal and coating performance.

Proper removal of excessive residues contributes to several advantages in the coating process, with the most important advantage being that it is a prerequisite for monomolecular layer formation. Without thorough removal of excess coating solution, true monomolecular layers cannot be achieved, as residual material would create irregular, multi-molecular deposits that compromise the precise layering structure. Only when excess material is completely removed can the remaining molecules self-organize into the desired monomolecular arrangement through electrostatic interactions. Additional advantages include:
1. Improved coating uniformity: By removing excess coating material, the residue removal process helps ensure a more even distribution of the coating across the medical textile substrate surface.
2. Enhanced layer definition: Thorough removal of residues between coating applications allows for better definition and separation of individual coating layers in multilayer structures.
3. Optimized functional properties: Removing excess coating material helps maintain the intended thickness and composition of each coating layer, which may be crucial for achieving specific functional properties such as antimicrobial activity or controlled drug release.
4. Reduced material waste: Efficient removal and recovery of excess coating solution can contribute to more economical use of coating materials and reduced environmental impact.
5. Improved coating adhesion: Removal of unbound coating molecules may enhance the adhesion between the applied coating and the substrate surface, potentially improving the durability and longevity of the coated medical textile.

The residue removal process may be integrated into a continuous coating system, allowing for efficient processing of long lengths of medical textile substrate. The positioning and design of the rinsing baths or flushing devices within the coating apparatus may be optimized to ensure consistent and thorough residue removal throughout the coating process.

In summary, the residue removal process using rinsing baths or flushing devices plays a vital role in achieving high-quality, uniform coatings on medical textile substrates. By effectively removing excessive residues between coating applications, this process contributes to the overall performance and functionality of the coated medical textiles produced using the present invention.

According to a particularly preferred embodiment the method according to the invention comprises applying the coating solution A to the textile substrate by immersing the textile substrate in a first coating bath containing the coating solution A for 10 to 30 seconds, removing excessive residues of the coating solution A from the textile substrate by immersing the textile substrate in a first rinsing bath containing a buffer, applying the coating solution B to the textile substrate by immersing the textile substrate web in a second coating bath containing coating solution B for 10 to 30 seconds, and removing excessive residues of the coating solution B from the textile substrate by immersing the textile substrate in a second rinsing bath containing a buffer. This cycle of treatment steps is repeated between 10 to 30 times. Finally, the textile substrate is dried using a drying device and collected. Preferably, the individual coating and rinsing steps are performed essentially as described in WO 2017/191110 A1.

The present invention solves the problems mentioned above by providing an apparatus for coating a medical textile substrate with multilayer coatings, particularly antimicrobial multilayer coatings. The apparatus enables the application of alternating layers quickly, easily, and cost-effectively on an industrial scale.

An apparatus for coating a textile substrate web comprises a donor device for providing the textile substrate web, a first coating bath or a first spray-coating device, a first rinsing bath or a first flushing device, a second coating bath or a second spray-coating device, a second rinsing bath or a second flushing device, a drying device, and a receiving device for collecting the coated textile substrate web. The apparatus may optionally include a plurality of diverter rollers or diverter bars for guiding the textile substrate web through the process.

The apparatus includes an unwinder roller for providing the textile substrate web. This unwinder roller allows for continuous feeding of the textile substrate into the coating process.

A first coating bath is included in the apparatus for applying a first coating solution to the textile substrate web. The first coating bath may be filled with a coating solution A. Alternatively, a first spray-coating device may be used to apply the coating solution A to the textile substrate web.

Following the first coating application, the apparatus includes a first rinsing bath or first flushing device. This component serves to remove excess coating solution from the textile substrate web, ensuring a clean and uniform coating layer. When using a flushing device instead of a rinsing bath, the textile substrate is subjected to controlled streams of buffer solution that effectively wash away unbound coating molecules while minimizing water consumption.

This component serves to remove excess coating solution from the textile substrate web, ensuring a clean and uniform coating layer.

The apparatus further comprises a second coating bath. The second coating bath may be filled with a coating solution B, or a second spray-coating device may be used to apply coating solution B to the textile substrate web. This allows for the application of a second, different coating layer to the textile substrate web.

After the second coating application, a second rinsing bath or a second flushing device is included in the apparatus. This component removes excess coating solution B from the textile substrate web, maintaining the integrity of the second coating layer. When using a flushing device instead of a rinsing bath, the textile substrate is subjected to controlled streams of buffer solution that effectively wash away unbound coating molecules while potentially reducing water consumption compared to full immersion rinsing.

A drying device is incorporated into the apparatus to set the applied coatings and prepare the substrate for subsequent processing or collection. The drying device may be a forced air dryer with adjustable temperature controls, an infrared panel heater with variable intensity settings, a contact heat roller, a vacuum drying chamber, a steam-heated cylinder, or a combination system employing multiple drying technologies in sequence. The choice of drying technology can be tailored to the specific requirements of the coating materials and textile substrate.

The apparatus includes a rewinder roller for collecting the coated textile substrate web. This rewinder roller allows for continuous collection of the processed textile substrate. The rewinder roller is designed to maintain consistent tension during the winding process, typically incorporating adjustable torque control to accommodate different textile weights and coating thicknesses. This tension control is critical for preventing coating damage or deformation in the final collected product. The rewinder roller may include features such as variable speed control synchronized with the overall processing line speed, edge alignment mechanisms to ensure proper web tracking, and diameter sensors that automatically adjust winding parameters as the roll diameter increases. For applications requiring precise tension profiles, the rewinder roller may incorporate dancer rollers or load cells that provide feedback to the tension control system.

Throughout the apparatus, a plurality of diverter rollers may optionally be included. These components guide the textile substrate web through the various processing stations, ensuring proper handling and exposure to the coating and rinsing solutions.

The apparatus may also include web tension control and/or measurement devices positioned at various points along the processing path. These devices help maintain proper tension of the textile substrate web throughout the coating process, contributing to uniform coating application and preventing damage to the substrate.

In some configurations, the apparatus may include additional coating baths filled with coating solution A or B, as well as additional rinsing baths. These extra processing stations allow for the application of multiple alternating layers of coatings, enhancing the functional properties of the coated textile substrate.

The apparatus is designed to enable continuous processing of the textile substrate web as it moves from the unwinder roller to the rewinder roller. This continuous process facilitates high-volume production while maintaining consistent coating quality. The arrangement and sequence of components within the apparatus allow for the efficient application of multiple coating layers, rinsing steps, and drying, resulting in a coated textile substrate web with desired properties for various applications, including medical textiles with antimicrobial characteristics.

The geometry of the coating baths plays a role in the coating process efficiency. The bath length is greater than the bath width and the web width of the textile substrate web. The bath height is greater than the bath width. This vertical orientation allows for efficient use of space while providing sufficient contact time between the substrate and the coating solutions. The narrow width of the baths helps to minimize the volume of coating solution required, potentially reducing waste and improving cost-efficiency while ensuring uniform coating application across the textile substrate web.

A coating apparatus may be configured with various arrangements of components to optimize the coating process for different textile substrates and coating requirements. In one example, a coating apparatus includes an unwinder roller, a first coating bath, a first rinsing bath, a second coating bath, a second rinsing bath, a drying device, and a rewinder roller arranged in a linear configuration. This arrangement allows for a straightforward processing path and may be suitable for applications where space constraints are not a primary concern.

In another example, a coating apparatus may utilize a more compact design by arranging the processing components in a vertical or stacked configuration. This arrangement may include multiple levels of coating and rinsing baths, with the textile substrate web guided between levels using diverter rollers. The vertical configuration may offer advantages in terms of reduced floor space requirements and potentially improved energy efficiency due to gravity-assisted fluid flow between processing stages.

A coating apparatus may also incorporate modular design elements, allowing for easy reconfiguration or expansion of the coating process. This modular approach may include interchangeable coating and rinsing bath units that can be added, removed, or rearranged to accommodate different coating sequences or substrate requirements. The modular design may enhance the versatility of the coating apparatus, enabling adaptation to various coating formulations and production needs.

In some examples, a coating apparatus may integrate in-line monitoring and control systems to enhance process consistency and quality. These systems may include sensors for measuring coating thickness, web tension, solution concentrations, and other critical parameters. The data from these sensors may be fed into a central control system that can make real-time adjustments to processing conditions, ensuring optimal coating performance and uniformity throughout long production runs.

A coating apparatus may also feature advanced drying technologies to optimize the setting and curing of applied coatings. For instance, a combination drying system may incorporate initial forced air drying to remove bulk moisture, followed by infrared heating for precise temperature control during the final curing stage. This multi-stage drying approach may allow for better management of coating properties and potentially reduce energy consumption compared to single-technology drying methods.

In summary, the coating apparatus described in this invention provides a versatile and efficient platform for applying multilayer coatings to medical textile substrates. The apparatus design allows for continuous processing, precise control of coating application, and flexibility in accommodating various coating materials and substrate types. The incorporation of features such as modular components, advanced drying technologies, and in-line monitoring systems enhances the capabilities of the coating apparatus, enabling the production of high-quality coated medical textiles with tailored functional properties.

The present invention solves the problems mentioned above by providing a method for coating medical textile substrates with multilayer coatings that are particularly advantageous for use in medical dressings and bandages. This coating method offers several benefits for medical applications, including enhanced antimicrobial properties, controlled drug release capabilities, and improved wound healing characteristics.

The multilayer coatings produced by this method may provide extended antimicrobial activity, which is a particular advantage for medical dressings and bandages. This prolonged antimicrobial effect may result from the layered structure of the coating.

The extended antimicrobial activity may be especially beneficial in wound care applications, where sustained protection against microbial colonization is crucial for proper healing. Medical dressings and bandages incorporating these multilayer coatings may require less frequent changes, potentially reducing the risk of contamination during dressing replacement procedures and improving patient comfort.

Additionally, the layered structure may allow for the incorporation of different types of antimicrobial agents in separate layers. This approach may provide broad-spectrum protection against various microorganisms and may help mitigate the development of antimicrobial resistance. The combination of immediate and sustained antimicrobial release may offer comprehensive protection throughout the healing process.

The extended antimicrobial activity of these coatings may also contribute to improved cost-effectiveness in healthcare settings by potentially reducing the frequency of dressing changes and lowering the risk of infection-related complications. This sustained protective effect may be particularly valuable for patients with compromised immune systems or in situations where frequent wound care is challenging.

Medical dressings and bandages produced using the coated textile substrates of this invention may exhibit superior performance compared to conventional uncoated materials. The multilayer coating structure allows for the incorporation of various therapeutic agents, such as antibiotics, growth factors, or anti-inflammatory compounds. These agents can be incorporated into the coating layers at concentrations ranging from 0.01% to 1.0% w/v, providing a wide range of treatment options for different medical conditions.

One of the key advantages of this coating method for medical applications is the ability to modulate the release profiles of therapeutic agents. By adjusting the number of coating layers applied to the textile substrate, typically between 4 and 20 layers, the release kinetics of incorporated drugs or bioactive compounds can be fine-tuned. Additionally, the ionic strength of the coating solutions, which can range from 0.05 M to 0.5 M NaCl, can be adjusted to further control the release properties of the therapeutic agents.

The multilayer coating structure may provide sustained release of antimicrobial agents, helping to prevent infection in wound care settings. This sustained antimicrobial activity may reduce the frequency of dressing changes, potentially improving patient comfort and reducing healthcare costs. The coated medical textiles may also demonstrate excellent moisture management properties, which can help maintain an optimal wound healing environment.

For applications requiring controlled drug delivery, the coated medical textiles may offer significant advantages. The layer-by-layer coating approach allows for the strategic placement of different therapeutic agents within the coating structure. For example, anti-inflammatory compounds may be incorporated into the outer layers for immediate release, while antibiotics may be placed in deeper layers for sustained, long-term delivery. This tailored drug release profile may enhance treatment efficacy and reduce the risk of systemic side effects associated with conventional drug administration methods.

The coating method may also allow for the development of advanced wound dressings with multiple functionalities. For instance, a coated medical textile may combine antimicrobial properties with the ability to promote tissue regeneration through the controlled release of growth factors. This multifunctional approach may accelerate wound healing and reduce the risk of complications.

The versatility of the coating method enables the creation of medical dressings and bandages tailored to specific wound types or medical conditions. By adjusting the coating composition, number of layers, and incorporated therapeutic agents, medical textiles can be optimized for various applications such as chronic wound management, burn treatment, or post-surgical care.

In summary, the coated medical textiles produced using this invention offer significant advantages for medical dressing and bandage applications. The ability to incorporate and control the release of therapeutic agents, combined with enhanced antimicrobial properties and moisture management capabilities, may lead to improved patient outcomes and more effective wound care strategies.

The present invention utilizes various materials in the coating process to create functional multilayer coatings on medical textile substrates. The primary coating materials include Poly-L-arginine, Poly-ε-Lysine, and hyaluronan, which are selected for their biocompatibility and ability to form stable electrostatic interactions.

Coating solution A typically contains Poly-L-arginine or Poly-ε-Lysine or a mixture of Poly-L-arginine and Poly-ε-Lysine as the primary coating molecule. Poly-L-arginine is a cationic polypeptide composed of L-arginine amino acid residues linked by peptide bonds, with the chemical formula (C6H14N4O2)n and characterized by positively charged guanidinium groups in the side chains. Poly-ε-Lysine is a cationic homopolymer composed of L-lysine amino acid residues linked by peptide bonds between the carboxyl and epsilon-amino groups, with the chemical formula (C6H14N2O2)n and characterized by positively charged amino groups in the side chains.

The concentration of Poly-L-arginine or Poly-ε-Lysine in coating solution A can be 0.1 to 2.0 mg/mL. This concentration range allows for effective formation of monomolecular layers while maintaining process efficiency. Lower concentrations may result in incomplete layer coverage, while higher concentrations may lead to excessive material usage without significant improvement in coating quality.

Coating solution A can have a pH of 6.0 to 7.5. This pH range ensures that the Poly-L-arginine or Poly-ε-Lysine remains positively charged, promoting electrostatic interactions with the substrate and subsequent layers. The pH can be adjusted using appropriate buffers or dilute acids/bases as needed.

Coating solution B typically contains hyaluronan (also known as hyaluronic acid) as the primary coating molecule. Hyaluronan is an anionic, non-sulfated glycosaminoglycan distributed widely throughout connective, epithelial, and neural tissues, with the chemical formula (C14H21NO11)n and characterized by negatively charged carboxyl groups along its polymer chain.

The Hyaluronan contained in coating solution B can have a molecular weight of 1.0 to 1.5 MDa. This molecular weight range provides a good balance between solution viscosity, layer formation properties, and biological activity. Higher molecular weight Hyaluronan may result in thicker layers but may also increase solution viscosity, potentially affecting the coating process.

The concentration of Hyaluronan in coating solution B can be 0.1 to 2.0 mg/mL, more preferably 0.5 mg/ml. This concentration range allows for effective layer formation while maintaining process efficiency. As with Poly-L-arginine or Poly-ε-Lysine, lower concentrations may result in incomplete layer coverage, while higher concentrations may not provide significant additional benefits.

Coating solution B can have a pH of 6.0 to 7.5. This pH range ensures that the Hyaluronan remains negatively charged, promoting electrostatic interactions with the previously deposited Poly-L-arginine or Poly-ε-Lysine layer. The pH can be adjusted using appropriate buffers or dilute acids/bases as needed.

The solvent used for coating solutions A and B can be a buffer, in particular a NaCl-Tris buffer. In one example, said NaCl-Tris buffer comprises 150 mM NaCl and 10 mM Tris at a pH 7.4. This buffer provides a controlled environment for the dissolution of the polyelectrolytes and helps maintain the desired pH of the coating solutions, ensuring consistent performance throughout the coating process.

The coating solutions can have a viscosity ranging from 0.25 cP to 1000 cP, with a preferred range of 0.30 cP to 2.00 cP. This viscosity range facilitates proper coating application and penetration into the textile structure. Lower viscosities may allow for better penetration into porous substrates, while higher viscosities may result in thicker coating layers.

The temperature of the coating baths can be maintained within a range of 10°C to 80°C, with a preferred range of 20°C to 60°C. This temperature range helps optimize the coating process for different coating materials and substrate types. Higher temperatures may increase the rate of layer formation but may also affect the stability of certain coating materials.

Variants and alternatives to the primary coating materials can be considered for specific applications. For example Chitosan and alginate: Chitosan is a cationic polysaccharide derived from chitin, while alginate is an anionic polysaccharide extracted from brown algae. This combination may be used for applications requiring enhanced wound healing properties.

The molecular weight of the polyelectrolytes can be varied to adjust the coating properties. Higher molecular weight polymers generally result in thicker layers and may provide enhanced durability, while lower molecular weight polymers may offer improved penetration into porous substrates.

For applications requiring enhanced antimicrobial activity, small amounts of antimicrobial agents such as silver nanoparticles, quaternary ammonium compounds, or other biocompatible antimicrobial substances may be incorporated into the coating solutions. These additives can provide additional protection against a broader spectrum of microorganisms.

The ionic strength of the coating solutions can be adjusted to fine-tune the layer formation process. Higher ionic strengths generally lead to thinner layers due to charge screening effects, while lower ionic strengths may result in thicker layers. The ionic strength can be adjusted by adding small amounts of salts such as sodium chloride or by modifying the buffer concentration.

In some examples, therapeutic agents such as antibiotics, growth factors, or anti-inflammatory compounds can be incorporated into the coating solutions at concentrations of 0.01-1.0% w/v. The release profiles of these agents can be modulated by adjusting the number of coating layers (typically 4-20) and the ionic strength of the coating solutions (0.05-0.5 M NaCl).

The choice of materials, their concentrations, and process parameters can be optimized based on the specific requirements of the intended application, such as antimicrobial efficacy, biocompatibility, durability, or controlled release properties. For antimicrobial applications, the concentration of Poly-L-arginine in coating solution A may be adjusted between 0.1-2.0 mg/mL to achieve different levels of antimicrobial potency, with higher concentrations generally providing enhanced activity against gram-negative bacteria.

The flexibility of this coating method allows for a wide range of material combinations and process variations to achieve desired functional properties in the coated textile substrates. By carefully selecting and optimizing the coating materials and process parameters, medical textiles with tailored properties can be produced for various applications in wound care, drug delivery, and infection control.

Features of any of the examples or embodiments outlined above may be combined to create additional examples or embodiments without losing the intended effect. It should be understood that the description of an embodiment or example provided above is by way of example only, and various modifications could be made by one skilled in the art. Furthermore, one skilled in the art will recognise that numerous further modifications and combinations of various aspects are possible. Accordingly, the described aspects are intended to encompass all such alterations, modifications, and variations that fall within the scope of the appended claims.

### FIGURES

Non-limiting and non-exhaustive examples are described with reference to the following figures. The figures illustrate various aspects of an apparatus and method for coating the textile substrate web with multiple layers. The figures show:
FIG. 1 Schematic side view of an apparatus (100) for coating a textile substrate web (15). It shows a complete processing sequence from an unwinder roll (1) to a rewinder roll (12), including multiple coating baths, rinsing baths, a squeezing device, and a drying unit. Diverting rollers guide the substrate throughout the process.
FIG. 2 Schematic side view of an alternative configuration of the coating apparatus (200). It illustrates a streamlined process with fewer coating stages compared to FIG. 1, detailing the path of the textile substrate web through various treatment baths and highlighting the strategic placement of diverting rollers.
FIG. 3 Schematic side view of a simplified version of the coating apparatus (300). It focuses on the core components of the coating process, showing the arrangement of coating and rinsing baths, as well as the crucial role of diverting rollers in maintaining proper substrate tension and guidance.
FIG. 4 Flowchart of the method for coating the textile substrate. It delineates each step of the process from unwinding to rewinding, providing a clear, sequential representation of the coating procedure and emphasizing the order and significance of each treatment stage.
FIG. 5 Orthogonal view of a coating bath illustrating the dimensional parameters.
FIG. 6 Orthogonal view of the textile substrate web illustrating the dimensional parameters.

### DETAILED DESCRIPTION OF THE FIGURES

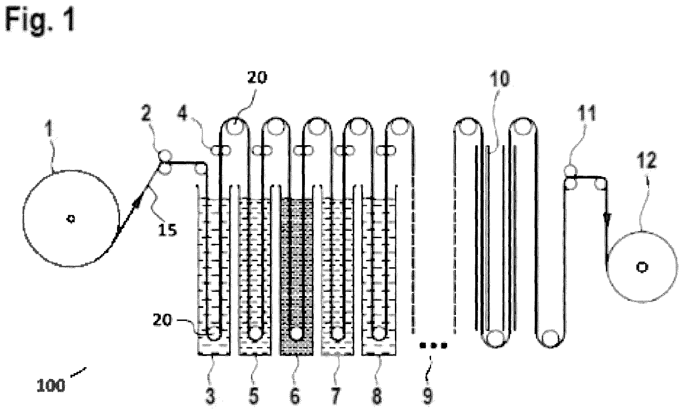

FIG. 1 provides a detailed schematic side view of an embodiment of the apparatus (100) for coating a textile substrate web (15), illustrating the complete processing sequence from unwinder roll (1) to rewinder roll (12). The figure showcases multiple processing stations including a first coating bath (3) containing coating solution A, a squeezing device (4) with a pair of rollers, a first rinsing bath (5) filled with buffer, a second coating bath (6) containing coating solution B, a second rinsing bath (7) filled with buffer, a further coating bath (8) filled with coating solution A, and additional coating and rinsing baths (9). Accordingly reference (9) may represent a series of multiple baths arranged in a row. This configuration allows for the application of additional coating layers and subsequent rinsing steps beyond the initial coating sequence. The number of baths included in this series may vary depending on the desired coating thickness, complexity of the multilayer structure, or specific requirements of the final coated textile product. By incorporating multiple baths in succession, the apparatus can facilitate the creation of more complex coating structures with a higher number of alternating layers without the need for rewinding or reprocessing the textile substrate web. This arrangement may enhance production efficiency and enable the creation of coatings with advanced functional properties through the strategic layering of different coating solutions.

The apparatus also includes web tension control and/or measurement devices (2, 11) positioned after the unwinder roll (1) and before the rewinder roll (12), respectively. A drying device (10), which may be an oven, infrared field, heat blower or combination thereof, is positioned near the end of the processing sequence. Throughout the apparatus, multiple diverting rollers or bars (20) are strategically positioned to guide the textile substrate web (15) through each processing station, ensuring proper web handling and exposure to the various coating and rinsing solutions.

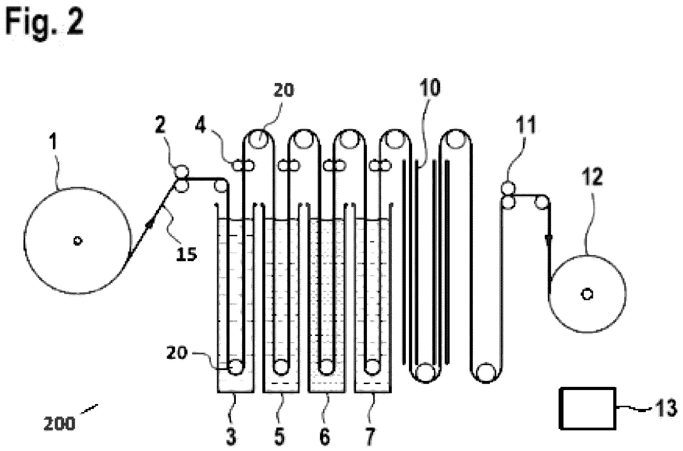

FIG. 2 presents a comprehensive schematic side view of an alternative configuration of the coating apparatus (200), emphasizing a streamlined process with fewer coating stages compared to FIG. 1. This figure meticulously details the path of the textile substrate web (15) through various treatment baths, beginning at the unwinder roll (1) and terminating at the rewinder roll (12). The textile substrate web (15) first passes through a web tension control and/or measurement device (2) before entering the first coating bath (3) containing coating solution A. After exiting the first coating bath (3), the web proceeds through a squeezing device (4) equipped with a pair of rollers that remove excess coating solution. The web then continues through a first rinsing bath (5) filled with buffer solution, followed by a second coating bath (6) containing coating solution B, and subsequently a second rinsing bath (7) filled with buffer solution. Before collection on the rewinder roll (12), the web passes through a drying device (10), which may be an oven, infrared field, heat blower, or combination thereof, and another web tension control and/or measurement device (11). The figure highlights the strategic placement of multiple diverting rollers or bars (20) throughout the apparatus, which are positioned within each bath and at critical transition points to ensure optimal substrate handling, maintain proper web tension, and facilitate smooth directional changes as the web navigates through the coating process. A controller (13) is also shown, which coordinates the operation of the various components to ensure synchronized processing.

The coating process may be repeated multiple times to build up additional layers on the textile substrate web. In some aspects, the method may involve cycling the substrate through the coating and rinsing steps several times to achieve the desired number of alternating layers. The number of repetitions may be adjusted based on the specific requirements of the final coated textile product.

By repeating the process, multiple bilayers consisting of coating A and coating B may be deposited on the substrate surface. In some cases, this repetition may allow for the creation of complex multilayer structures with enhanced functional properties. The ability to control the number of coating cycles may provide flexibility in tailoring the thickness, composition, and performance characteristics of the final coated textile.

The repeated application of alternating layers may contribute to improved coating durability, as each additional layer may reinforce the overall coating structure. In some implementations, the multilayer coating formed through repeated cycles may exhibit synergistic effects between the different coating materials, potentially leading to enhanced antimicrobial efficacy, improved barrier properties, or other desired functionalities.

The apparatus may be configured to accommodate multiple passes of the textile substrate web through the coating and rinsing stations. In some configurations, this may involve redirecting the web back through the same processing sequence, while in other arrangements (see Figure 1), additional coating and rinsing baths may be incorporated in series to facilitate continuous multilayer deposition without the need for rewinding.

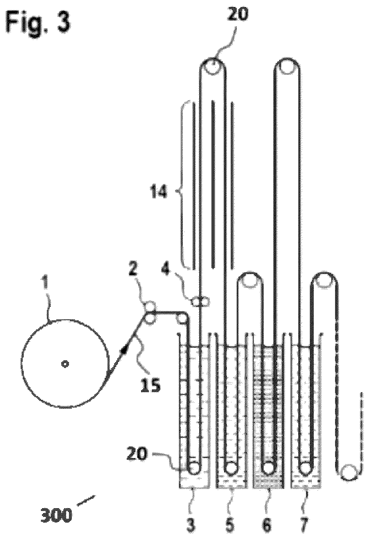

FIG. 3 offers an in-depth schematic side view of a simplified version of the coating apparatus (300), focusing on the core components essential for the basic multilayer coating process. This figure elucidates the arrangement of coating and rinsing baths in a compact configuration, beginning with an unwinder roll (1) that serves as the donor device for providing the textile substrate web (15). After passing through a web tension control and/or measurement device (2), the textile substrate web (15) enters a first coating bath (3) containing coating solution A, where it is immersed to apply the initial coating layer. Upon exiting the first coating bath (3), the web proceeds through a squeezing device (4) with a pair of rollers that apply pressure to remove excess coating solution, ensuring a more uniform coating thickness. The web then continues to a first rinsing bath (5) filled with buffer solution, which removes any residual unbound coating material from the substrate surface. Following this rinsing step, the web enters a second coating bath (6) containing coating solution B, where a second, different coating layer is applied. After the second coating application, the web passes through a second rinsing bath (7) filled with buffer solution to remove excess coating solution B. Throughout this processing sequence, the figure illustrates the crucial role of multiple diverting rollers or bars (20) strategically positioned within each bath and at transition points between processing stations. These diverting elements serve multiple critical functions: they maintain proper substrate tension to prevent sagging or wrinkling, guide the web along a predetermined path through each processing bath, ensure consistent immersion depth in the coating and rinsing solutions, facilitate directional changes with minimal stress on the substrate, and enable a compact apparatus design by allowing vertical or serpentine web paths. The precise positioning of these diverting rollers or bars (20) is essential for achieving uniform coating application and maintaining the integrity of the textile substrate throughout the coating process.

Additionally, a drying device (14) located between the first coating bath and the first rinsing bath may be included to provide intermediate drying when needed.

The drying device (14) located between the first coating bath (3) and the first rinsing bath (5) may serve several important functions in the coating process. This intermediate drying step may help control the moisture content of the textile substrate web after the initial coating application. By partially or fully drying the substrate before it enters the first rinsing bath (5), the drying device (14) may enhance the stability of the first coating layer and potentially improve its adhesion to the textile surface.

In some cases, the drying device (14) may also help prevent excessive dilution of the rinsing bath by removing excess coating solution from the substrate. This may lead to more efficient use of the rinsing solution and potentially reduce contamination between processing stages. The presence of this drying device may allow for greater flexibility in adjusting process parameters, as it provides an additional point of control in managing the coating application and rinsing steps.

The drying device (14) may utilize various technologies such as forced air, infrared heating, or contact heat rollers, depending on the specific requirements of the coating materials and textile substrate. The intensity and duration of the drying process at this stage may be adjusted to optimize the overall coating performance and efficiency of the subsequent rinsing step.

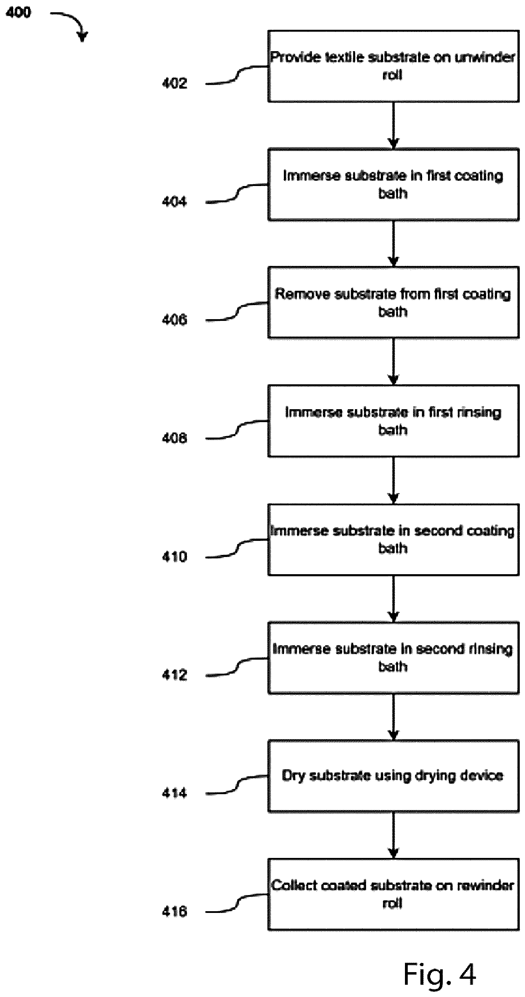

FIG. 4 illustrates a detailed flowchart of the method for coating the textile substrate, delineating each step of the process from unwinding to rewinding. This figure provides a clear, sequential representation of the coating procedure, emphasizing the order and significance of each treatment stage in the overall coating process.

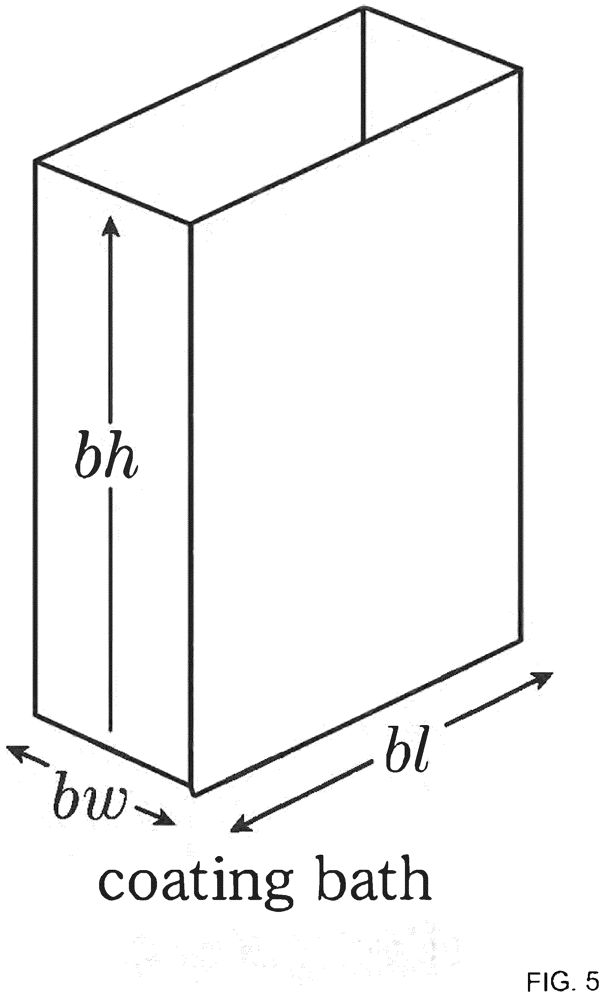

FIG. 5 illustrates an orthogonal view of a coating bath. The figure shows the dimensional parameters of the bath structure, including the bath height (bh), bath width (bw), and bath length (bl). The bath is depicted as a rectangular container with an open top.

The bath length (bl) extends along the horizontal axis and represents the longest dimension of the bath. The bath width (bw) represents the shorter horizontal dimension of the bath. The bath height (bh) represents the vertical dimension of the bath from its base to its upper edge.

The dimensions are arranged such that the bath length (bl) is greater than the bath width (bw), and the bath height (bh) is greater than the bath width (bw). These proportions create a vertically-oriented, elongated bath structure suitable for immersing textile substrates during the coating process.

The figure includes dimensional arrows and labels indicating the bath height (bh), bath width (bw), and bath length (bl), providing a clear representation of the bath's proportional relationships and spatial configuration.

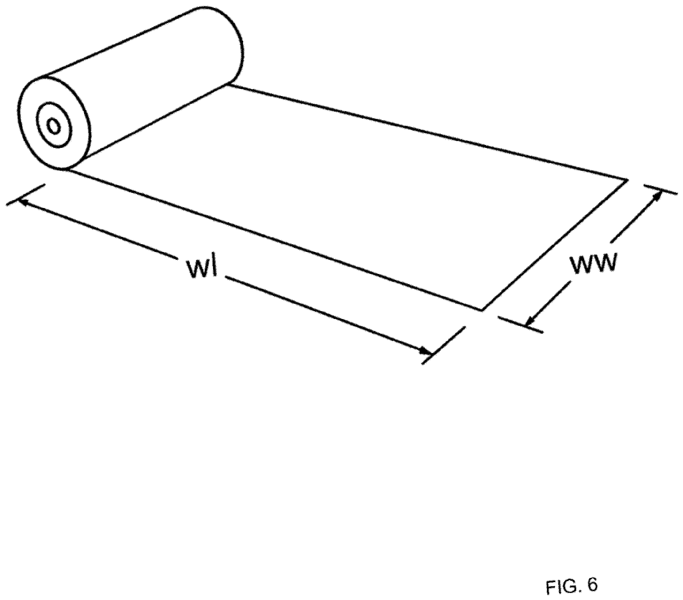

FIG. 6 illustrates an orthogonal view of the textile substrate web. The figure shows the dimensional parameters of the web, including the web length (wl) and web width (ww). The textile substrate web is depicted as a rectangular shape with a roll at one end.

The web length (wl) extends along the horizontal axis and represents the longer dimension of the web. The web width (ww) represents the shorter horizontal dimension of the web, measured perpendicular to the web length. A typical web width is 110 cm. Typical web length are in the range of 50 to 200 m.

The figure includes dimensional arrows and labels indicating the web length (wl) and web width (ww), providing a clear representation of the web's dimensional parameters. The roll portion at one end may indicate how the textile substrate web can be stored and dispensed during processing.

This orthogonal view helps visualize the key dimensions of the textile substrate web that are relevant to the coating process. Understanding these parameters may be important for optimizing the coating apparatus design and ensuring proper handling of the substrate throughout the coating procedure.

Common reference numerals are used throughout the figures to indicate similar features.

## Claims

1. A method for coating a medical textile substrate (15) with at least two alternating layers of a coating, the method comprising:
step i) providing the textile substrate (15);
step ii) applying a coating solution A comprising coating molecules A to the textile substrate (15);
step iii) removing excessive residues of the coating solution A from the textile substrate (15);
step iv) applying a coating solution B comprising coating molecules B to the textile substrate (15);
step v) removing excessive residues of the coating solution B from the textile substrate (15);
step vi) optionally drying the textile substrate web (15) using a drying device (14);
and wherein the coating molecules A and the coating molecules B can attract each other through electrostatic attraction.

2. The method according to claim 1, wherein the coating is an antimicrobial effective coating.

3. The method according to claim 1 or 2, wherein the coating molecules A are Poly-L-arginine or Poly-ε-Lysine or a mixture of Poly-L-arginine and Poly-ε-Lysine,
and wherein the coating molecules B are hyaluronan.

4. The method according to claim 3, wherein a monomolecular layer of Poly-L-arginine or a monomolecular Poly-ε-Lysine or a monomolecular layer comprising a mixture of Poly-L-arginine and Poly-ε-Lysine is formed by applying the coating solution A to the textile substrate (15) and removing excessive residues of coating solution A from the textile substrate (15); and
wherein a monomolecular hyaluronan layer is formed by applying the coating solution B to a section of the textile substrate (15) and removing excessive residues of coating solution B from the section of the textile substrate (15).

5. The method according to any one of claims 1 to 4, further comprising repeating cycles comprising the steps ii) to v) between 2 and 100 times, preferably between 4 and 30 times, to obtain between 4 and 200, preferably between 8 and 60 alternating coating layers on the textile substrate web (15).

6. The method according to any one of claims 1 to 5, wherein applying each of the coating solution A and the coating solution B to the textile substrate web (15) is performed between 3 seconds and 300 seconds, in particular between 10 seconds and 30 seconds.

7. The method according to any one of claims 1 to 6, wherein the textile substrate (15) comprises one or more of woven textiles, knitted textiles, nonwoven textiles, and textile nets.

8. The method according to any one of claims 1 to 7, wherein the textile substrate (15) has an open-pored structure with a pore diameter of 0.1 to 5 mm.

9. The method according to any one of claims 1 to 8, wherein applying the coating solution A to the textile substrate web (15) in step ii) is performed by immersing the textile substrate (15) in a first coating bath (3) containing the coating solution A or by applying the coating solution A to the textile substrate (15) by the use of a first spray-coating device, and/or wherein applying the coating solution B to the textile substrate (15) in step iv) is performed by immersing the textile substrate (15) in a second coating bath (6) containing the coating solution B or by applying the coating solution B to the section of the textile substrate (15) by the use of a second spray-coating device.

10. The method according to any one of claims 1 to 9, wherein the textile substrate (15) is provided as a textile substrate web (15) having a length of at least 500 cm.

11. The method according to claim 10, wherein the textile substrate web (15) is provided on an unwinder roller (1) or a donor container and wherein the textile substrate web (15) is received on a rewinder roller (12) or in a recipient container, and wherein the textile substrate web (15) is transported by continuously moving the textile substrate web (15) from the unwinder roller (1) or donor container to the rewinder roller (12) or recipient container (12) by the use of diverter devices, in particular diverter rollers (20) or diverter bars (20).

12. The method according to claim 11, wherein applying the coating solution A to the textile substrate web in step ii) is performed by applying the coating solution A to a section of the textile substrate web (15) by immersing the section of the textile substrate web (15) in a first coating bath (3) containing the coating solution A or by applying the coating solution A to the section of the textile substrate web (15) by the use of a first spray-coating device, and/or wherein applying the coating solution B to the textile substrate web in step iv) is performed by applying the coating solution B to the section of the textile substrate web (15) by immersing the section of the textile substrate web (15) in a second coating bath (6) containing the coating solution B or by applying the coating solution B to the section of the textile substrate web (15) by the use of a second spray-coating device.

13. The method according to claim 12, wherein removing excessive residues of the coating solution A from the section of the textile substrate web (15) in step iii) is performed by immersing the section of the textile substrate web (15) in a first rinsing bath (5) or by flushing the section of the textile substrate web (15) in a first flushing device, and/or wherein removing excessive residues of the coating solution B from the section of the textile substrate web (15) in step v) is performed by immersing the section of the textile substrate web (15) in a second rinsing bath (7) or by flushing the section of the textile substrate web (15) in a second flushing device.

14. An apparatus (100, 200, 300) for coating a textile substrate web (15), the apparatus comprising:
an unwinder roller or a donor container (1) for providing the textile substrate web (15);
a first coating bath (3) or a first spray-coating device;
a first rinsing bath (5) or a first flushing device;
a second coating bath (6) or a second spray-coating device;
a second rinsing bath (7) or a second flushing device;
a drying device (10);
a rewinder roller (12) or recipient container for collecting the coated textile substrate web (15); and
a plurality of diverter rollers or diverter bars for guiding the textile substrate web (15) through the process,
wherein the apparatus (100, 200, 300) is configured to perform the method according to any one of claims 10 to 13.

15. A medical dressing or bandage comprising a coated textile layer, which textile layer is cut out of a coated textile substrate (15) or textile substrate web (15) produced by a method according to any one of claims 1 to 13.
